# EUROPÄISCHE PATENTANMELDUNG

(11) **EP 1 430 887 A1**
(43) Veröffentlichungstag der Anmeldung: **23.06.2004**
(21) Anmeldenummer: 04006349.7
(22) Anmeldetag: 28.09.2001
(51) Int. Cl.: A61K 9/00, A61K 31/46

(54) **Tiotropium-haltige Inhalationspulver**

(30) Priorität: 12.10.2000 DE 10050635
(62) Teilanmeldung aus: 01969789.5
(71) Anmelder: Boehringer Ingelheim Pharma GmbH & Co.KG, 55216 Ingelheim am Rhein (DE)
(72) Erfinder: Bechtold-Peters, Karoline, 88400 Biberach-Rissegg (DE); Boeck, Georg, 55122 Mainz (DE); Dörr, Rolf, 55270 Ober-Olm (DE); Walz, Michael, 55411 Bingen (DE)

(57) **Zusammenfassung**

Die Erfindung betrifft Tiotropium enthaltende pulverförmige Zubereitungen für die Inhalation, Verfahren zu deren Herstellung sowie deren Verwendung zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

## Beschreibung

Die Erfindung betrifft Tiotropium enthaltende pulverförmige Zubereitungen für die Inhalation, Verfahren zu deren Herstellung sowie deren Verwendung zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

### Hintergrund der Erfindung

Tiotropiumbromid ist aus der Europäischen Patentanmeldung EP 418 716 A1 bekannt und weist die folgende chemische Struktur auf:

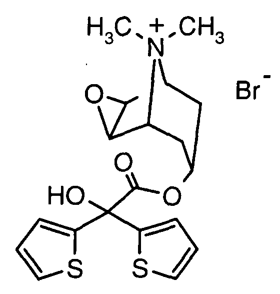

Tiotropiumbromid stellt ein hoch wirksames Anticholinergikum mit langanhaltender Wirkdauer dar, welches zur Therapie von Atemwegserkrankungen, insbesondere von COPD (chronic obstructive pulmonary disease = chronisch obstruktive Lungenerkrankung) und Asthma Verwendung finden kann. Unter Tiotropium ist das freie Ammoniumkation zu verstehen.

Bei der Behandlung vorstehender Erkrankungen bietet sich die inhalative Applikation des Wirkstoffs an. Neben der inhalativen Applikation von broncholytisch wirksamen Verbindungen in Form von Dosieraerosolen und Lösungen zur Inhalation kommt der Applikation von wirkstoffhaltigen Inhalationspulvern besondere Bedeutung zu.

Bei Wirkstoffen, die eine besonders hohe Wirksamkeit aufweisen, sind pro Einzeldosis zur Erzielung des therapeutisch erwünschten Effekts nur geringe Mengen des Wirkstoffs erforderlich. In solchen Fällen ist es notwendig, zur Herstellung des Inhalationspulvers den Wirkstoff mit geeigneten Hilfsstoffen zu verdünnen. Aufgrund des hohen Anteils an Hilfsstoff werden die Eigenschaften des Inhalationspulvers maßgeblich durch die Wahl des Hilfsstoffs beeinflußt. Bei der Wahl des Hilfsstoffs kommt dessen Korngröße eine besondere Bedeutung zu. Je feiner der Hilfsstoff desto schlechter sind in der Regel dessen Fließeigenschaften.

Gute Fließeigenschaften sind allerdings Voraussetzung für eine hohe Dosiergenauigkeit bei der Abfüllung und Abteilung der einzelnen Präparatedosen, wie etwa bei der Herstellung von Kapseln (Inhaletten) zur Pulverinhalation oder der Dosierung eines Einzelhubes durch den Patienten vor der Anwendung eines Mehrdosisinhalators. Des weiteren ist die Korngröße des Hilfsstoffs von großer Bedeutung für das Entleerungsverhalten von Kapseln in einem Inhalator bei der Anwendung. Es hat sich ferner gezeigt, daß die Korngröße des Hilfsstoffs starken Einfluß auf den inhalierbar ausgebrachten Wirkstoffanteil des Inhalationspulvers hat. Unter inhalierbarem bzw. inhalierfähigem Wirkstoffanteil werden die Teilchen des Inhalationspulvers verstanden, die beim Inhalieren mit der Atemluft tief in die Verästelungen der Lunge transportiert werden. Die hierzu erforderliche Teilchengröße liegt zwischen 1 und 10µm, vorzugsweise unter 6 µm.

Es ist Aufgabe der Erfindung, ein Tiotropium-haltiges Inhaltionspulver bereitzustellen, welches bei guter Dosiergenauigkeit (betreffend die pro Kapsel herstellerseitig abgefüllte Menge an Wirkstoff und Pulvermischung wie auch die pro Kapsel durch den Inhalationsvorgang ausgebrachte und lungengängige Wirkstoffmenge) und geringer chargenweisen Variabilität die Applikation des Wirkstoffs mit hohem inhalierfähigem Anteil erlaubt. Es ist ferner Aufgabe der vorliegenden Erfindung, ein Tiotropium-haltiges Inhaltionspulver bereitzustellen, welches ein gutes Entleerungsverhalten der Kapseln gewährleistet, sollte es z.B. mittels eines Inhalators, wie er beispielsweise in der WO 94/28958 beschrieben wird, am Patienten oder invitro über einen Impaktor oder Impinger, zur Anwendung gelangen.

Daß Tiotropium, insbesondere Tiotropiumbromid, bereits in sehr geringen Dosen eine hohe therapeutische Wirksamkeit aufweist, stellt weitere Anforderungen an ein mit hoher Dosiergenauigkeit einzusetzendes Inhalationspulver. Aufgrund der geringen, zur Erzielung des therapeutischen Effekts erforderlichen Konzentration des Wirkstoffs im Inhalationspulver, muß ein hohes Maß an Homogenität der Pulvermischung und eine geringe Schwankung im Dispergierverhalten von Charge zu Charge Pulverkapsel gewährleistet werden. Die Homogenität der Pulvermischung wie auch gering schwankende Dispergiereigenschaften tragen entscheidend dazu bei, daß die Freigabe des inhalierfähigen Anteils des Wirkstoffs reproduzierbar in gleichbleibend hohen Mengen und somit möglichst geringer Variabilität erfolgt.

Dementsprechend ist es ferner Aufgabe der vorliegenden Erfindung, ein Tiotropium-haltiges Inhaltionspulver bereitzustellen, welches durch eine hohes Maß an Homogenität und Gleichförmigkeit der Dispergierbarkeit gekennzeichnet ist. Ferner zielt die vorliegende Erfindung auf die Bereitstellung eines Inhalationspulvers, welches die Applikation des inhalierfähigen Wirkstoffanteils bei möglichst geringer Variabilität erlaubt.

### Detaillierte Beschreibung der Erfindung

Überraschenderweise wurde gefunden, daß die eingangs genannten Aufgaben durch die nachstehend beschriebenen, erfindungsgemäßen pulverförmigen Zubereitungen für die Inhalation (Inhalationspulver) gelöst werden.

Dementsprechend zielt die vorliegende Erfindung auf Inhalationspulver enthaltend 0,04 bis 0,8% Tiotropium im Gemisch mit einem physiologisch unbedenklichen Hilfsstoff, dadurch gekennzeichnet, daß der Hilfsstoff aus einem Gemisch von gröberem Hilfsstoff mit einer mittleren Teilchengröße von 15 bis 80µm und feinerem Hilfststoff mit einer mittleren Teilchengröße von 1 bis 9µm besteht, wobei der Anteil von feinerem Hilfsstoff an der Gesamthilfsstoffmenge 1 bis 20% beträgt. Erfindungsgemäß bevorzugt sind Inhalationspulver, die 0,08 bis 0,64%, besonders bevorzugt 0,16 bis 0,4% Tiotropium enthalten.

Unter Tiotropium ist das freie Ammoniumkation zu verstehen. Als Gegenion (Anion) kommen Chlorid, Bromid, Iodid, Methansulfonat, para-Toluolsulfonat oder Methylsulfat in Betracht. Von diesen Anionen ist das Bromid bevorzugt. Dementsprechend betrifft die vorliegende Erfindung bevorzugt Inhalationspulver, die zwischen 0,048 und 0,96% Tiotropiumbromid enthalten. Erfindungsgemäß von besonderem Interesse sind Inhalationspulver, die 0,096 bis 0,77%, besonders bevorzugt 0,19 bis 0,48% Tiotropiumbromid enthalten.

Das in den erfindungsgemäßen Inhalationspulvern bevorzugt enthaltene Tiotropiumbromid kann bei der Kristallisation Lösungsmittelmoleküle mit einschließen. Bevorzugt werden zur Herstellung der erfindungsgemäßen Tiotropiumhaltigen Inhalationspulver die Hydrate des Tiotropiumbromids, besonders bevorzugt das Tiotropiumbromid-monohydrat eingesetzt. Dementsprechend betrifft die vorliegende Erfindung Inhaltionspulver, die zwischen 0,05 und 1% Tiotropiumbromidmonohydrat enthalten. Erfindungsgemäß von besonderem Interesse sind Inhalationspulver, die 0,1 bis 0,8%, besonders bevorzugt 0,2 bis 0,5% Tiotropiumbromid-monohydrat enthalten.

Die erfindungsgemäßen Inhalationspulver sind bevorzugt dadurch gekennzeichnet, daß der Hilfsstoff aus einem Gemisch von gröberem Hilfsstoff mit einer mittleren Teilchengröße von 17 bis 50µm, besonders bevorzugt von 20 bis 30µm und feinerem Hilfststoff mit einer mittleren Teilchengröße von 2 bis 8µm, besonders bevorzugt von 3 bis 7µm besteht. Dabei wird unter der mittleren Teilchengröße im hier verwendeten Sinne der 50%-Wert aus der Volumenverteilung gemessen mit einem Laserdiffraktometer nach der Trockendispersionsmethode verstanden. Bevorzugt sind Inhalationspulver bei denen der Anteil von feinerem Hilfsstoff an der Gesamthilfsstoffmenge 3 bis 15%, besonders bevorzugt 5 bis 10% beträgt.

Bei den im Rahmen der vorliegenden Erfindung genannten prozentualen Angaben, handelt es sich stets um Gewichtsprozent.

Wird im Rahmen der vorliegenden Erfindung auf die Bezeichnung Gemisch Bezug genommen, so ist hierbei stets eine Mischung zu verstehen, die durch Mischen zuvor klar definierter Komponenten erhalten wurde. Entsprechend sind beispielsweise als Hilfsstoffgemisch aus gröberen und feineren Hilfsstoffanteilen nur solche Gemische zu verstehen, die durch Mischen einer gröberen Hilfsstoffkomponente mit einer feineren Hilfsstoffkomponente erhalten werden.

Die gröberen und feineren Hilfsstoffanteile können aus dem chemisch gleichen oder aus chemisch verschiedenen Substanzen bestehen, wobei Inhalationspulver, bei denen der gröbere Hilfsstoffanteil und der feinere Hilfsstoffanteil aus der selben chemischen Verbindung bestehen bevorzugt sind.

Als physiologisch unbedenkliche Hilfsstoffe, die zur Darstellung der erfindungsgemäßen Inhalationspulver zur Anwendung gelangen können seine beispielsweise genannt Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciulcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die erfindungsgemäßen Inhalationspulver können beispielsweise mittels Inhalatoren appliziert werden, die eine einzelne Dosis aus einem Vorrat mittels einer Meßkammer (z.B. gemäß US 4570630A) oder über andere apparative Vorrichtungen (z.B. gemäß DE 36 25 685 A) dosieren. Vorzugsweise werden die erfindungsgemäßen Inhalationspulver allerdings in Kapseln abgefüllt (zu sogenannten Inhaletten), die in Inhalatoren wie beispielsweise in der WO 94/28958 beschrieben, zur Anwendung gelangen.

Soll das erfindungsgemäße Inhalationspulver im Sinne der vorstehend gennannten bevorzugten Anwendung in Kapseln (Inhaletten) abgefüllt werden, bieten sich Füllmengen von 3 bis 10mg, bevorzugt von 4 bis 6mg Inhalationspulver pro Kapsel an. Diese enthalten dann zwischen 1,2 und 80µg Tiotropium. Bei einer bevorzugten Füllmenge von 4 bis 6 mg Inhalationspulver pro Kapsel sind zwischen 1,6 und 48µg, bevorzugt zwischen 3,2 und 38,4µg, besonders bevorzugt zwischen 6,4 und 24µg Tiotropium pro Kapsel enthalten. Ein Gehalt von beispielsweise 18µg Tiotropium entspricht dabei einem Gehalt von etwa 21,7µg Tiotropiumbromid.

Folglich enthalten Kapseln mit einer Füllmenge von 3 bis 10mg Inhaltionspulver erfindungsgemäß bevorzugt zwischen 1,4 und 96,3µg Tiotropiumbromid. Bei einer bevorzugten Füllmenge von 4 bis 6 mg Inhalationspulver pro Kapsel sind zwischen 1,9 und 57,8µg, bevorzugt zwischen 3,9 und 46,2µg, besonders bevorzugt zwischen 7,7 und 28,9µg Tiotropiumbromid pro Kapsel enthalten. Ein Gehalt von beispielsweise 21,7µg Tiotropiumbromid entspricht dabei einem Gehalt von etwa 22,5µg Tiotropiumbromid-monohydrat.

Folglich enthalten Kapseln mit einer Füllmenge von 3 bis 10mg Inhaltionspulver bevorzugt zwischen 1,5 und 100µg Tiotropiumbromid-monohydrat. Bei einer bevorzugten Füllmenge von 4 bis 6 mg Inhalationspulver pro Kapsel sind zwischen 2 und 60µg, bevorzugt zwischen 4 und 48µg, besonders bevorzugt zwischen 8 und 30µg Tiotropiumbromid-monohydrat pro Kapsel enthalten.

Die erfindungsgemäßen Inhalationspulver sind entsprechend der der vorliegenden Erfindung zugrunde liegenden Aufgabe gekennzeichnet durch ein hohes Maß an Homogenität im Sinne der Einzeldosierungsgenauigkeit. Diese liegt in einem Bereich von < 8% , bevorzugt < 6% , besonders bevorzugt < 4%.

Die erfindungsgemäßen Inhalationspulver sind gemäß der nachfolgend beschriebenen Vorgehensweise erhältlich:
Nach Einwaage der Ausgangsmaterialien erfolgt zunächst die Fertigung der Hilfsstoffmischung aus den definierten Fraktionen des gröberen Hilfsstoffs und des feineren Hilfsstoffs. Anschließend erfolgt die Herstellung der erfindungsgemäßen Inhalationspulver aus der Hilfsstoffmischung und dem Wirkstoff. Soll das Inhalationspulver mittels Inhaletten in hierzu geeigneten Inhalatoren appliziert werden, schließt sich der Herstellung der Inhalationspulver die Fertigung der pulverhaltigen Kapseln an.

Bei den nachstehend beschriebenen Herstellverfahren werden die genannten Komponenten in den Gewichtsanteilen eingesetzt, wie sie in den zuvor beschriebenen Zusamensetzungen der erfindungsgemäßen Inhalationspulver beschrieben wurden.
Die Herstellung der erfindungsgemäßen Inhaltionspulver erfolgt durch Mischen der gröberen Hilfsstoffanteile mit den feineren Hilfststoffanteilen und andschließendem Mischen der so erhaltenen Hilfsstoffgemische mit dem Wirkstoff.
Zur Herstellung der Hilfsstoffmischung werden die gröberen und feineren Hilfsstoffanteile in einen geeigneten Mischbehälter eingebracht. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der gröbere Hilfsstoff vorgelegt und anschließend der feinere Hilfsstoffanteil in den Mischbehälter eingebracht. Bevorzugt erfolgt bei diesem Mischverfahren die Zugabe der beiden Komponenten portionsweise, wobei ein Teil des gröberen Hilfsstoffs zunächst vorgelegt und anschließend abwechselnd feinerer und gröberer Hilfsstoff zugegeben wird. Besonders bevorzugt ist bei der Herstellung der Hilfsstoffmischung das abwechselnde, schichtweise Einsieben der beiden Komponenten. Vorzugsweise erfolgt das Einsieben der beiden Komponenten abwechselnd in je 15 bis 45, besonders bevorzugt in je 20 bis 40 Schichten. Der Mischvorgang der beiden Hilfsstoffe kann bereits während der Zugabe der beiden Komponenten erfolgen. Vorzugsweise wird allerdings erst nach dem schichtweisen Einsieben der beiden Bestandteile gemischt.

Nach Herstellung der Hilfsstoffmischung werden diese und der Wirkstoff in einen geeigneten Mischbehälter eingebracht. Der verwendete Wirkstoff weist eine mittlere Teilchengröße von 0,5 bis 10µm, vorzugsweise von 1 bis 6µm, besonders bevorzugt von 2 bis 5µm auf. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird die Hilfsstoffmischung vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist bei der Herstellung der Hilfsstoffmischung das abwechselnde, schichtweise Einsieben der beiden Komponenten. Vorzugsweise erfolgt das Einsieben der beiden Komponenten abwechselnd in je 25 bis 65, besonders bevorzugt in je 30 bis 60 Schichten. Der Mischvorgang der Hilfsstoffmischung mit dem Wirkstoff kann bereits während der Zugabe der beiden Komponenten erfolgen. Vorzugsweise wird allerdings erst nach dem schichtweisen Einsieben der beiden Bestandteile gemischt.

Die so erhaltene Pulvermischung kann gegebenenfalls erneut ein- oder mehrfach über einen Siebgranulator gegeben und jeweils anschließend einem weiteren Mischvorgang unterworfen werden.

Ein Aspekt der vorliegenden Erfindung betrifft ein Tiotropium-haltiges Inhaltionspulver, welches gemäß der voranstehend beschriebenen Vorgehensweisen erhältlich ist.

Wird im Rahmen der vorliegenden Erfindung der Begriff Wirkstoff verwendet, so ist dies als Bezugnahme auf Tiotropium zu verstehen. Eine Bezugnahme auf Tiotropium, welches das freie Ammoniumkation darstellt, entspricht erfindungsgemäß einer Bezugnahme auf Tiotropium in Form eines Salzes (Tiotropium-Salz), welches ein Anion als Gegenion enthält. Als' im Rahmen der vorliegenden Erfindung einzetzbare Tiotropium-Salze sind die Verbindungen zu verstehen, die neben Tiotropium als Gegenion (Anion) Chlorid, Bromid, Iodid, Methansulfonat, para-Toluolsulfonat oder Methylsulfat enthalten. Im Rahmen der vorliegenden Erfindung ist von allen Tiotropiumsalzen das Tiotropiumbromid bevorzugt. Bezugnahmen auf Tiotropiumbromid sind im Rahmen der vorliegenden Erfindung stets als Bezugnahmen auf alle möglichen amorphen und kristallinen Modifikationen des Tiotropiumbromids zu verstehen. Diese können beispielsweise in der kristallinen Struktur Lösemittelmoleküle mit einschließen. Von allen kristallinen Modifikationen des Tiotropiumbromids sind erfindungsgemäß diejenigen, die Wasser mit einschließen (Hydrate) bevorzugt. Besonders bevorzugt ist im Rahmen der vorliegenden Erfindung das Tiotropiumbromid-monohydrat einsetzbar.

Für die Herstellung der erfindungsgemäßen Fromulierungen ist es zunächst erforderlich, Tiotropium in für die pharmazeutische Anwendung verwendbarer Form bereitzustellen. Bevorzugt wird dazu Tiotropiumbromid, welches wie in der EP 418 716 A1 offenbart, hergestellt werden kann, einem weiteren Kristallisationsschritt unterworfen. Je nach Wahl der Reaktionsbedingungen und des Lösemittels werden dabei unterschiedliche Kristallmodifikationen erhalten. Diese Modifikationen lassen sich beispielsweise mittels DSC (Differential Scanning Calorimetry) unterscheiden. Nachstehende Tabelle faßt die mittels DSC bestimmten Schmelzpunkte unterschiedlicher Kristallmodifikationen des Tiotropiumbromids in Abhängigkeit vom Lösemittel zusammen.

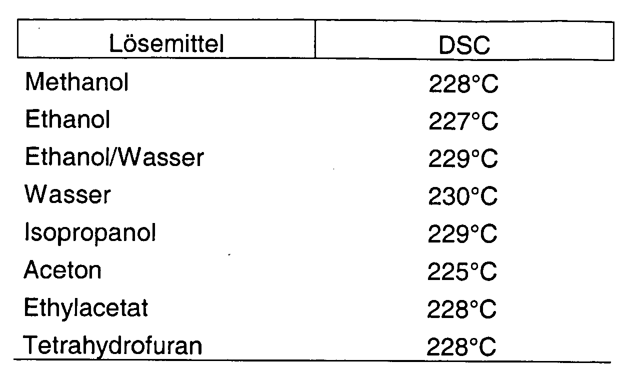

Für die Zwecke der Herstellung der erfindungsgemäßen Formulierung hat sich das Tiotropiumbromid-monohydrat als besonders geeignet erwiesen. Das DSCdiagramm des Tiotropiumbromid-monohydrats weist zwei charakteristische Signale auf. Das erste, relativ breite, endotherme Signal zwischen 50-120°C ist auf die Entwässerung des Tiotropiumbomid-monohydrats zur wasserfreien Form zurückzuführen. Das zweite, relativ scharfe, endotherme Maximum bei 230 ± 5°C ist dem Schmelzen der Substanz zuzuordnen.Diese Daten wurden mittels eines Mettler DSC 821 erhalten und mit dem Mettler Software-Paket STAR ausgewertet.
Diese Daten, wie auch die anderen in der voranstehenden Tabelle genannten Werte wurden bei einer Heizrate von 10 K/min erhoben.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Ausgangsmaterialien

In den nachfolgenden Beispielen wird als gröberer Hilfsstoff Lactose-Monohydrat (200M) verwendet. Dieser kann beispielsweise von der Firma DMV International, 5460 Veghel/NL unter der Produktbezeichnung Pharmatose 200M bezogen werden.

In den nachfolgenden Beispielen wird als feinerer Hilfsstoff Lactose-Monohydrat (5µ) verwendet. Dieser kann durch gängige Verfahren (Mikronisieren) aus Lactose-Monohydrat 200M erhalten werden. Lactose-Monohydrat 200M kann beispielsweise von der Firma DMV International, 5460 Veghel/NL unter der Produktbezeichnung Pharmatose 200M bezogen werden.

### Darstellung von Tiotropiumbromid-monohydrat:

In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromid-monohydrat (86 % d. Th.)

Das so erhaltene kristalline Tiotropiumbromid-monohydrat wird nach bekannten Verfahren mikronisiert, um den Wirkstoff in Form der mittleren Teilchengröße bereitzustellen, die den erfindungsgemäßen Spezifikationen entspricht.

Nachfolgend wird beschrieben, wie die Bestimmung der mittleren Teilchengröße der verschiedenen Bestandteile der erfindungsgemäßen Formulierung erfolgen kann.

### A) Partikelgrößenbestimmung von feinteiliger Lactose:

### Meßgerät und Einstellungen:

Die Bedienung der Geräte erfolgt in Übereinstimmung mit den Bedienungsanleitungen des Herstellers.

| | |
|---|---|
| Meßgerät | HELOS Laser-Beugungs-Spektrometer , (SympaTec) |
| Dispergiereinheit | RODOS Trockendispergierer mit Saugtrichter, (SympaTec) |
| Probenmenge | ab 100 mg |
| Produktzufuhr | Schwingrinne Vibri, Fa. Sympatec |
| Frequenz d. Vibrationsrinne | 40 bis 100 % ansteigend |
| Dauer der Probenzufuhr | 1 bis 15 sek. (im Fall von 100 mg) |
| Brennweite | 100 mm (Meßbereich: 0,9 - 175µm) |
| Meßzeit | ca. 15 s (im Fall von 100 mg) |
| Zykluszeit | 20 ms |
| Start/Stop bei | 1 % auf Kanal 28 |
| Dispergiergas | Druckluft |
| Druck | 3 bar |
| Unterdruck | maximal |
| Auswertemodus | HRLD |

### Probenvorbereitung / Produktzufuhr:

Mind. 100 mg der Prüfsubstanz werden auf einem Kartenblatt eingewogen.
Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut. Nach dem Start der Messung wird die Frequenz der Schwingrinne von ca. 40 % bis 100 % (gegen Ende der Messung) variiert. Die Zeit, in der jeweils die gesamte Probe zugeführt wird beträgt 10 bis 15 sek.

### B) Partikelgrößenbestimmung von Tiotropiumbromidmonohydrat, mikronisiert:

### Meßgerät und Einstellungen:

Die Bedienung der Geräte erfolgte in Übereinstimmung mit den Bedienungsanleitungen des Herstellers.

| | |
|---|---|
| Meßgerät | Laser-Beugungs-Spektrometer (HELOS), Sympatec |
| Dispergiereinheit | Trockendispergierer RODOS mit Saugtrichter,Sympatec |
| Probenmenge | 50 mg - 400 mg |
| Produktzufuhr | Schwingrinne Vibri, Fa. Sympatec |
| Frequenz d. Vibrationsrinne | 40 bis 100 % ansteigend |
| Dauer der Probenzufuhr | 15 bis 25 sek. (im Fall von 200 mg) |
| Brennweite | 100 mm (Meßbereich: 0,9 - 175 µm) |
| Meßzeit | ca. 15 s (im Fall von 200 mg) |
| Zykluszeit | 20 ms |
| Start/Stop bei | 1 % auf Kanal 28 |
| Dispergiergas | Druckluft |
| Druck | 3 bar |
| Unterdruck | maximal |
| Auswertemodus | HRLD |

### Probenvorbereitung / Produktzufuhr:

ca. 200 mg der Prüfsubstanz werden auf einem Kartenblatt eingewogen.
Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut.

Nach dem Start der Messung wird die Frequenz der Schwingrinne von ca. 40 % bis 100 % (gegen Ende der Messung) variiert. Die Zufuhr der Probe soll möglicht kontinuierlich sein. Die Produktmenge darf aber auch nicht zu groß sein damit eine ausreichende Dispergierung erreicht wird. Die Zeit, in der jeweils die gesamte Probe zugeführt wird beträgt für 200 mg z. B. ca. 15 bis 25 sek.

### C) Partikelgrößenbestimmung von Laktose 200M :

### Meßgerät und Einstellungen

Die Bedienung der Geräte erfolgte in Übereinstimmung mit den Bedienungsanleitungen des Herstellers.

| | |
|---|---|
| Meßgerät | Laser-Beugungs-Spektrometer (HELOS), Sympatec |
| Dispergiereinheit | Trockendispergierer RODOS mit Saugtrichter, Sympatec |
| Probenmenge | 500 mg |
| Produktzufuhr | Vibrationsrinne Typ VIBRI , Sympatec |
| Frequenz d. Vibrationsrinne | 18 bis 100 % ansteigend |
| Brennweite (1) | 200 mm (Meßbereich: 1.8 - 350 µm) |
| Brennweite (2) | 500 mm (Meßbereich: 4.5 - 875 µm) |
| Meßzeit/Wartezeit | 10 s |
| Zykluszeit | 10 ms |
| Start/Stop bei | 1 % auf Kanal 19 |
| Druck | 3 bar |
| Unterdruck | maximal |
| Auswertemodus | HRLD |

### Probenvorbereitung / Produktzufuhr:

Ca. 500 mg der Prüfsubstanz werden auf einem Kartenblatt eingewogen. Mit einem weiteren Kartenblatt werden alle größeren Agglomerate zerstoßen. Das Pulver wird in der Trichter der Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne von 0 auf 40 % gesteigert bis sich ein kontinuierlicher Produktfluß einstellt. Danach wird auf eine Amplitude von ca. 18% reduziert. Gegen Ende der Messung wird die Amplitude auf 100% gesteigert.

### Apparatives

Zur Herstellung der erfindungsgemäßen Inhalationspulver können beispielsweise die folgenden Maschinen und Geräte Verwendung finden:

Mischbehälter bzw. Pulvermischer: Rhönradmischer 200 L; Typ: DFW80N-4; Hersteller: Firma Engelsmann, D-67059 Ludwigshafen.

Siebgranulator: Quadro Comil; Typ: 197-S; Hersteller: Firma Joisten & Kettenbaum, D-51429 Bergisch-Gladbach.

### Beispiel 1:

### 1.1: Hilfsstoffmischung:

Als gröbere Hilfsstoffkomponente werden 31,82 kg Lactose Monohydrat für Inhalationszwecke (200M) eingesetzt. Als feinere Hilfsstoffkomponente werden 1,68 kg Lactose Monohydrat (5µm) eingesetzt. In den daraus erhaltenen 33,5 kg Hilfsstoffmischung beträgt der Anteil der feineren Hilfsstoffkomponente 5%.

Über einen geeigneten Siebgranulator mit einem Sieb mit einer Maschenweite von 0,5 mm werden in einen geeigneten Mischbehälter ca. 0,8 bis 1,2 kg Lactose Monohydrat für Inhalationszwecke (200M) vorgelegt. Anschließend werden abwechselnd Lactose Monohydrat (5µm) in Portionen von ca. 0,05 bis 0,07 kg und Lactose Monohydrat für Inhalationszwecke (200M) in Portionen von 0,8 bis 1,2 kg schichtweise eingesiebt. Lactose Monohydrat für Inhalationszwecke (200M) und Lactose Monohydrat (5µm) werden in 31 bzw. in 30 Schichten (Toleranz: ±6 Schichten) zugegeben.

Die eingesiebten Bestandteile werden anschließend gemischt (Mischen: 900 Umdrehungen)

### 1.2: Endmischung:

Zur Herstellung der Endmischung werden 32,87 kg der Hilfsstoffmischung (1.1) und 0,13 kg Mikronisiertes Tiotropiumbromid-monohydrat eingesetzt. In den daraus erhaltenen 33,0 kg Inhalationspulver beträgt der Wirkstoffanteil 0.4%.

Über einen geeigneten Siebgranulator mit einem Sieb mit einer Maschenweite von 0,5 mm werden in einen geeigneten Mischbehälter ca. 1,1 bis 1,7 kg Hilfsstoffmischung (1.1) vorgelegt. Anschließend werden abwechselnd Tiotropiumbromid-monohydrat in Portionen von ca. 0,003 kg und Hilfsstoffmischung (1.1) in Portionen von 0,6 bis 0,8 kg schichtweise eingesiebt. Die Zugabe der Hilfststoffmischung und des Wirkstoffs erfolgt in 46 bzw. 45 Schichten (Toleranz: ± 9 Schichten).

Die eingesiebten Bestandteile werden anschließend gemischt (Mischen: 900 Umdrehungen). Die Endmischung wird noch zweimal über einen Siebgranulator gegeben und anschließend jeweils gemischt (Mischen: 900 Umdrehungen).

### Beispiel 2:

Mit der nach Beispiel 1 erhaltenen Mischung werden Inhalationskapseln (Inhaletten) der folgenden Zusammensetzung erhalten:

| | |
|---|---|
| Tiotropiumbromid-monohydrat | 0,0225 mg |
| Lactose Monohydrat (200 M) | 5,2025 mg |
| Lactose Monohydrat (5µm) | 0,2750 mg |
| Hartqelatinekapsel | 49,0 mg |
| Total | 54,5 mg |

### Beispiel 3:

Inhalationskapsel der Zusammensetzung:

| | |
|---|---|
| Tiotropiumbromid-monohydrat | 0,0225 mg |
| Lactose Monohydrat (200 M) | 4,9275 mg |
| Lactose Monohydrat (5µm) | 0,5500 mg |
| Hartgelatinekapsel | 49,0 mg |
| Total | 54,5 mg |

Das zur Herstellung der Kapsel erforderliche Inhalationspulver wurde in Analogie zu Beispiel 1 erhalten.

### Beispiel 4:

Inhalationskapsel der Zusammensetzung:

| | |
|---|---|
| Tiotropiumbromid-monohydrat | 0,0225 mg |
| Lactose Monohydrat (200 M) | 5,2025 mg |
| Lactose Monohydrat (5µm) | 0,2750 mg |
| Polyethylenkapsel | 100,0 mg |
| Total | 105,50 mg |

Das zur Herstellung der Kapsel erforderliche Inhalationspulver wurde in Analogie zu Beispiel 1 erhalten.

Im Sinne der vorliegenden Erfindung ist unter mittlerer Teilchengröße der Wert in µm zu verstehen, an dem 50% der Teilchen aus der Volumenverteilung eine kleinere oder die gleiche Partikelgröße besitzen im Vergleich zum angegebenen Wert. Zur Bestimmung der Summenverteilung der Partikelgrößenverteilung wird als Meßmethode Laserdiffraktion/Trockendispergierung angewendet.

## Patentansprüche

1. Inhalationspulver enthaltend 0,04 bis 0,8% Tiotropium im Gemisch mit einem physiologisch unbedenklichen Hilfsstoff, **dadurch gekennzeichnet, daß** der Hilfsstoff aus einem Gemisch von gröberem Hilfsstoff mit einer mittleren Teilchengröße von 15 bis 80µm und feinerem Hilfststoff mit einer mittleren Teilchengröße von 1 bis 9 µm besteht, wobei der Anteil von feinerem Hilfsstoff an der Gesamthilfsstoffmenge 1 bis 20% beträgt.

2. Inhaltionspulver nach Anspruch 1, **dadurch gekennzeichnet, daß** das Tiotropium in Form seines Chlorids, Bromids, lodids, Methansulfonats, para-Toluolsulfonats oder Methylsulfats vorliegt.

3. Inhaltionspulver nach Anspruch 1 oder 2, dadruch gekennzeichnet, daß es zwischen 0,048 und 0,96% Tiotropiumbromid enthält.

4. Inhaltionspulver nach einem der Ansprüche 1, 2 oder 3, **dadurch gekennzeichnet, daß** es zwischen 0,05 und 1% Tiotropiumbromid-monohydrat enthält.

5. Inhalationspulver nach einem der Ansprüche 1, 2, 3 oder 4, **dadurch gekennzeichnet, daß** der Hilfsstoff aus einem Gemisch von gröberem Hilfsstoff mit einer mittleren Teilchengröße von 17 bis 50µm und feinerem Hilfststoff mit einer mittleren Teilchengröße von 2 bis 8µm besteht.

6. Inhalationspulver nach einem der Ansprüche 1, 2, 3, 4 oder 5, **dadurch gekennzeichnet, daß** der Anteil von feinerem Hilfsstoff an der Gesamthilfsstoffmenge 3 bis 15% beträgt.

7. Inhalationspulver nach einem der Ansprüche 1, 2, 3, 4, 5 oder 6, **dadurch gekennzeichnet, daß** das eingesetzte Tiotropium-Salz eine mittlere Teilchengröße von 0,5 bis 10µm aufweist.

8. Inhalationspulver nach einem der Ansprüche 1 bis 7, **dadurch gekennzeichnet, daß** als Hilfststoffe Monosaccharide, Disaccharide, Oligo- und Polysaccharide, Polyalkohole, Salze oder Mischungen dieser Hilfsstoffe miteinander Verwendung finden.

9. Inhalationspulver nach Anspruch 8, **dadurch gekennzeichnet, daß** als Hilfststoffe Glucose, Arabinose, Lactose, Saccharose, Maltose, Dextrane, Sorbit, Mannit, Xylit, Natriumchlorid, Calciulcarbonat oder Mischungen dieser Hilfsstoffe miteinander Verwendung finden.

10. Inhalationspulver nach Anspruch 9, **dadurch gekennzeichnet, daß** als Hilfststoffe Glucose oder Lactose oder Mischungen dieser Hilfsstoffe miteinander Verwendung finden.

11. Verfahren zur Herstellung der Inhaltionspulver nach einem der Ansprüche 1 bis 10, **dadurch gekennzeichnet, daß** in einem ersten Schritt die gröberen Hilfsstoffanteile mit denn feineren Hilfststoffanteilen gemischt werden und in einem anschließenden Schritt das so erhaltene Hilfsstoffgemisch mit dem Tiotropium-Salz gemischt wird.

12. Inhaltionspulver gemäß einem der Ansprüche 1 bis 10, erhältlich nach Anspruch 11.

13. Verwendung eines Inhaltionspulvers nach einem der Ansprüche 1 bis 10 oder nach Anspruch 12 als Arzneimittel.

14. Verwendung eines Inhaltionspulvers nach einem der Ansprüche 1 bis 10 oder nach Anspruch 12 zur Herstellung eines Arzneimittels zur Behandlung von Erkrankungen, in denen Tiotropium-Salze einen therapeutischen Nutzen entfalten können.

15. Verwendung nach Anspruch 14 zur Herstellung eines Arzneimittels zur Behandlung von Asthma oder COPD.

16. Verwendung eines Inhaltionspulvers nach einem der Ansprüche 1 bis 10 oder nach Anspruch 12 zur Herstellung einer Kapsel (Inhalette).

17. Kapsel (Inhalette), **gekennzeichnet durch** eine Füllmenge von 3 bis 10mg an Inhaltionspulver gemäß einem der Ansprüche 1 bis 10 oder nach Anspruch 12.

18. Kapsel (Inhalette) nach Anspruch 15, **dadurch gekennzeichnet, daß** sie zwischen 1,2 und 80µg Tiotropium enthält.
